Europäisches Patentamt

(19) European Patent Office

Office européen des brevets

(11) Publication number: **0 243 803 B1**

# EUROPEAN PATENT SPECIFICATION

(12)

(45) Date of publication of patent specification :
**10.07.91 Bulletin 91/28**

(51) Int. Cl.⁵ : **A61B 17/32**

(21) Application number : **87105593.5**

(22) Date of filing : **15.04.87**

(54) Medical instrument for removing bone tissue.

(30) Priority : **02.07.86 US 881395**
**29.04.86 EP 86105889**

(43) Date of publication of application :
**04.11.87 Bulletin 87/45**

(45) Publication of the grant of the patent :
**10.07.91 Bulletin 91/28**

(84) Designated Contracting States :
**DE FR GB**

(56) References cited :
**US-A- 4 574 803**

(73) Proprietor : **Waldemar Link GmbH & Co**
**Barkhausenweg 10**
**W-2000 Hamburg 63 (DE)**

(72) Inventor : **Farley, Daniel K.**
**148 S. North West Highway**
**Barrington Illinois 60010 (US)**

(74) Representative : **Glawe, Delfs, Moll & Partner**
**Patentanwälte**
**Postfach 26 01 62 Liebherrstrasse 20**
**W-8000 München 26 (DE)**

## Description

### BACKGROUND OF THE INVENTION

This invention relates to forceps of the rongeur or bone cutting type and more particularly to a rongeur structure which prevents premature ware in the bone cutting surfaces.

It has long been an object in the art to provide simple, inexpensive forceps capable of easy disassembly and cleaning, and most importantly capable of safe and efficient operation. Prior patents issued in this art have proposed possible solutions. Typical of the prior art is the patent issued to De Vilbiss, U.S. Patent No. 1,040,523, which provides a lever mechanism comprising a handle having two sides arranged to pivot towards one another about a central axis. On extensions of these handle members there is provided a jaw mechanism comprising a first movable jaw arranged to travel within a slot defined within a second movable jaw. Bone seized between the jaws is pulled within the slot as it is cut.

A problem associated with this and other mechanisms in the art is that uncontrolled force is applied to the bone surface surrounding the cutting area, such that possible bone breakage and splintering may occur. If operating near the spinal cord, possible nerve damage may result. Further, bone chips cut from the bone structure are not well contained by the instrument and contamination of the wound may result. Finally, with the pivot placed between the jaw mechanism and the lever handles limited mechanical advantage is available, making the instrument tiring to use. Further, the large force applied to the bone may also be applied across the cutting surfaces which come into contact with each other at the end of the cutting stroke. Such large forces applied across contacting cutting surfaces cause unnecessary waring of the sharp edges.

The US-A 4 574 803 discloses a medical cutter particularly adapted for cutting and removing soft tissue, particularly usefull in laparoscopy. Though there is passing reference to cutting bone, the primary purpose of the known device is to cut soft tissue. It comprises a rigid barrel member having a cutting edge defined at one end thereof; a shaft member positioned coaxially within said barrel member and arranged for reciprocal motion therein, said shaft member having a plate member mounted at one extremity thereof transverse to said shaft member and defining, together with a depression in the shaft, a cavity proximate to said plate member; a second cutting edge being formed on said plate member; and lever means for reciprocally translating said shaft member within said barrel to cause said plate to reciprocate with respect to said barrel whereby tissue is trapped between said cutting edges, and to cause said cavity to reciprocate with respect to said barrel whereby severed tissue is captured within said cavity.

It is the primary aim of the present invention to provide an approved forceps or rongeur for cutting bone wherein the force applied between meeting cutting surfaces is decreased at the end of the cutting stroke in order to prevent ware thereto.

It is also an object of the present invention to eliminate uncontrolled forces in and about the cutting area to avoid possible bone breakage and splintering beyond the cutting area while utilizing structure which prevents excessive ware on the cutting surfaces.

It is also an object of the present invention to modify the mechanical leverage to provide improved mechanical advantage for the device and to provide better control during operation while utilizing structure which prevents excess ware on cutting surfaces located in the cutting area.

### SUMMARY OF THE INVENTION

These and other objects of the invention are accomplished in an improved forceps or rongeur as defined in claim 1. The instrument has inter alia a jaw mechanism comprised of a barrel member having a first cutting surface on one extremity thereof and having a shaft member located within and arranged for reciprocal motion within the barrel member. On the shaft member there is provided a plate member attached at one extremity and having a second cutting surface to trap bone against the first cutting surface of the barrel member. On the shaft member there is further provided a cavity arranged to slide within the barrel progressively as the cutting operation proceeds, gradually drawing severed bone within the capturing cavity, allowing for a cutting action rather than a crushing action, thereby relieving pressure on the plate member and thus reducing the possibility of breaking said member while cutting bone structures.

Other objects and advantages of the invention will become apparent upon reading the following detailed description and upon reference to the drawings.

### DESCRIPTION OF THE DRAWINGS

Fig. 1 is a side view partially cut away of a preferred embodiment of a rongeur of the present invention.

Fig. 2 is an enlarged perspective view of the distal end of the rongeur of Fig. 2 in operation.

Fig. 3 is a side view of the rongeur of Fig. 1.

Fig. 4 is a side view of a shaft member of the rongeur of Fig. 1.

Fig. 5 is a side view of the end of the barrel member of the rongeur of Fig. 1, showing the shaft member in its open position.

Fig. 6 is a side view of the end of the barrel member of the rongeur of Fig. 1, showing the shaft member in its closed position.

Fig. 7 is a cross-sectional view of the end of the shaft member taken along line 7.7. of Fig. 5.

Fig. 8 is a cross-sectional view of the end of the barrel member taken along line 8.8. of Fig. 5.

## DESCRIPTION OF THE PRFERRED EMBODIMENT

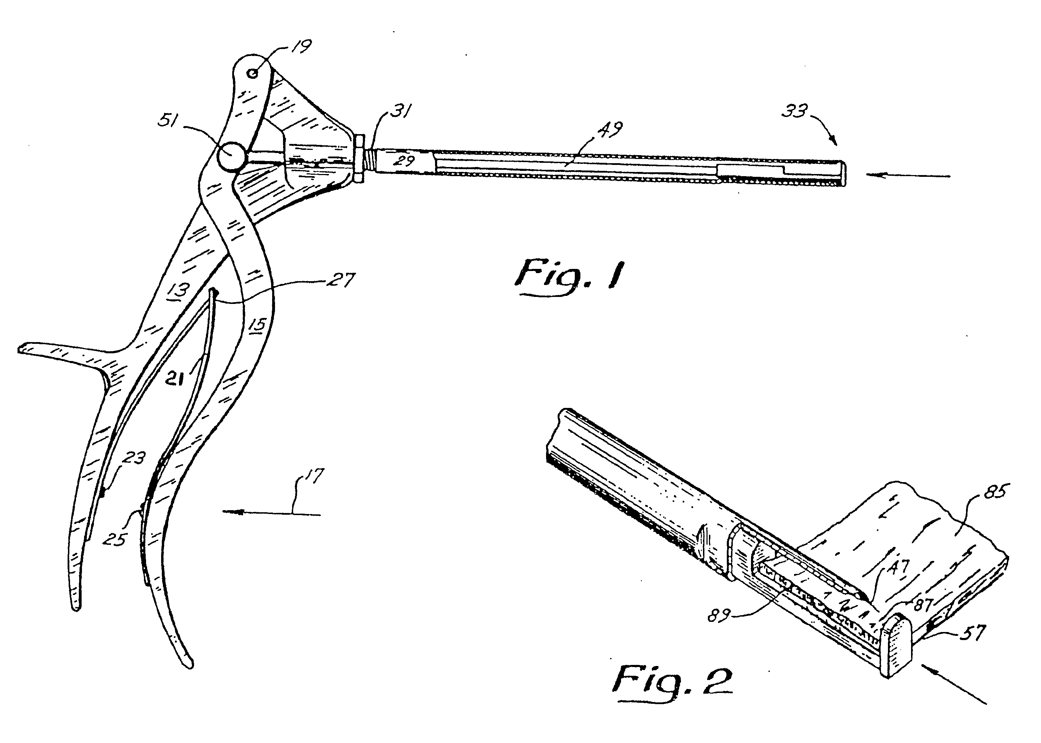

Referring to Fig. 1, a rongeur includes a pair of handle members 13, 15 arranged to be held within the human hand and pulled together in the direction of an arrow 17 much in the same manner as a pair of pliers. Handle members 13, 15 are arranged to pivot about an axis 19 and each handle is arranged as shown in the drawing to support and actuate a barrel member and a shaft cutting mechanism as more fully described below.

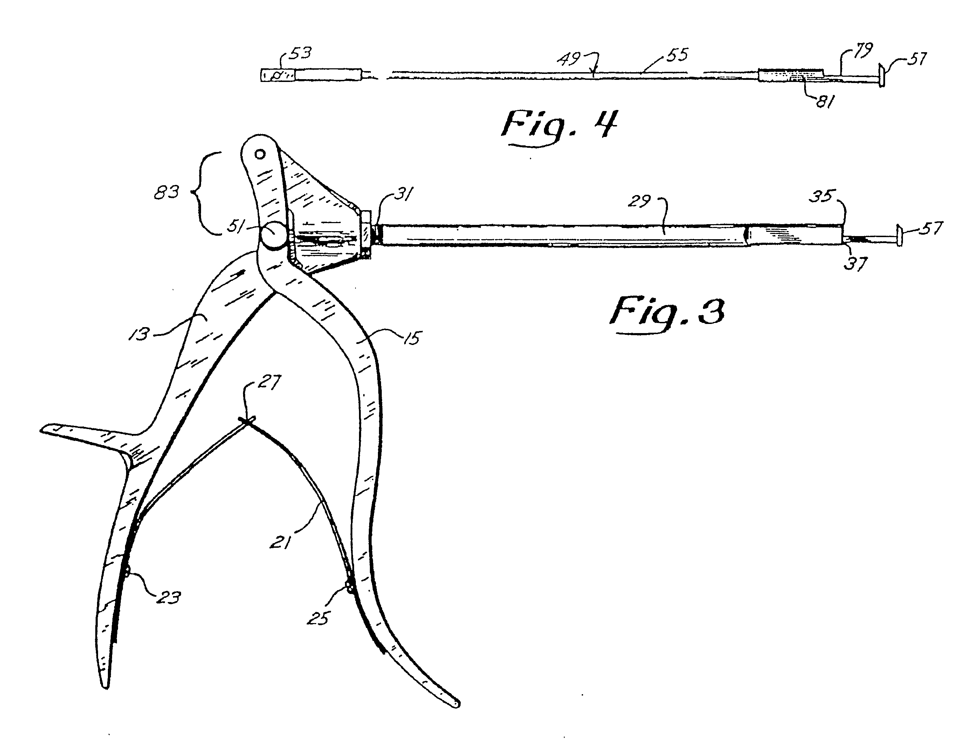

A squeezing force on the handle members closes the handles in the direction of arrow 17, and concurrently compresses an intermediate spring 21 located between the handle members. This generates a returning force to return the handles to their starting position as shown in Fig. 3. The return spring is typically formed of spring steel made of two distinct and separate members, each rigidly attached to respective individual handle members at lower extremity points 23, 25 respectively, while being hingedly interlocked at an intermediate contacting point 27.

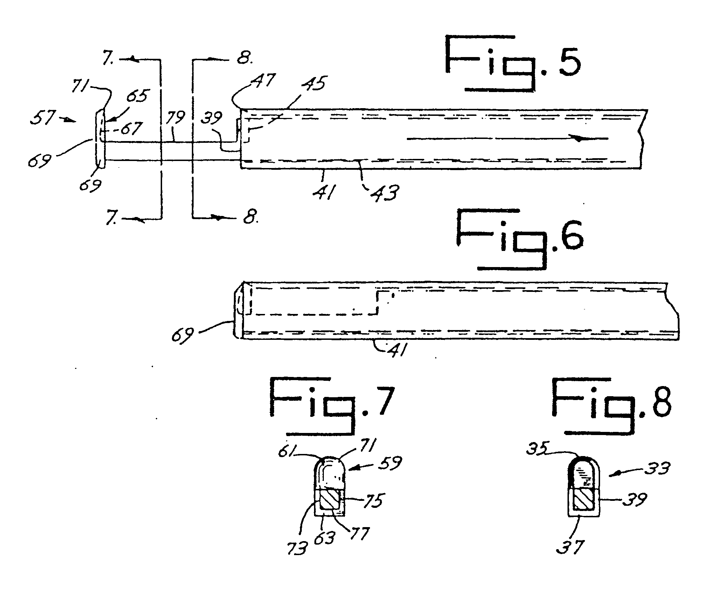

A barrel member 29 is rigidly attached by screw threads 31 to handle member 13. Barrel member 29 is formed of a cylindrical tube but having its distal end (as shown in Figs. 2 and 5) formed of a uniform cross-sectional shape being somewhat rectangular and formed of three straight sides and a protruding or curved fourth side, as shown in Fig. 8. Barrel member 29 has external threads 31 at its point of attachment to the handle, and has at its other extremity a work area 33 which includes a cutting surface 35 and a stop surface 37 (more clearly shown in Fig. 8). Cutting surface 35 and stop surface 37 are formed from the end edge 39 of the barrel member. The upper portion of end edge 39 is honed to form cutting surface 35 and the lower portion of the end edge 39 is formed flat to provide stop surface 37.

As shown in Fig. 5, barrel member 29 includes an outside face 41, forming the outer surface of the barrel member, and an inside face 43 spaced from the outside face and defining the containing area of the barrel member. Cutting surface 35 is formed from a bevel surface extending between the inside face (at 45) and the outside face of the barrel member. The intersection of the bevel surface and the outside face provides a cutting edge 47.

Mounted for reciprocal motion within the barrel there is provided a shaft member 49 (Fig. 1) pivotally attached at an axis 51 to handle member 15 and arranged to selectively protrude from the barrel at its other extremity. As shown in Fig. 4, shaft member 49 is comprised of a first attachment portion 53 for connection to the pivot mount at handle 15, and a central force transmission portion 55 for transmitting the force of the handle leverage mechanism remotely to the site of the bone cutting at the distal end of the barrel. Finally, there is a bone cutting and trapping portion comprised of a plate or foot 57 mounted transversely to the distal end of the shaft.

As shown in Figs. 5 and 7, plate member 57 includes a work area 59 which includes a cutting surface 61 and a stop surface 63. Cutting surface 61 and stop surface 63 are formed from the inside face 65 of the plate member which confronts end edge 39 of the barrel member.

The upper portion of inside face 65 includes a cavity 67 shaped into the plate member to form the cutting surface 61. An outside face 69 of the plate member forms the outer surface of the sides and end of the plate member. The surface defining cavity 67 intersects a portion of the outside face 69 forming a cutting edge 71.

The lower portion of inside face 65 seats the distal end of shaft member 49, which may be secured or formed integral to plate member 57. As shown in Fig. 7, the cross-sectional shape of the distal end of shaft member 49 is square and the end is secured to plate member 57 in a location to leave portions of inside face 65 at the sides 73, 75 and bottom 77 of the shaft member. These portions of inside face 65 are formed flat to provide stop surface 63.

Within shaft 49 there is provided a cavity portion 79 (see Figs. 4 and 5). Cavity portion 79 is formed by a flat depression or notch represented by a cut away portion of the shaft. The inside face of plate member 57 forms one end of cavity portion 79 and the other end is formed by a sealing portion 81 (Fig. 4). Sealing portion 81 is provided to close the gap between the barrel and the shaft as the shaft end is drawn into the barrel. This sealing portion acts to maintain the barrel free of debris and serves to eject bone fragment following a cutting sequence.

To improve the mechanical advantage of the forceps, increased leverage is provided by attaching the barrel and shaft between the handle pivot and the handle grasping portion where hand pressure is applied during the squeeze. By varying the distance 83 (Fig. 3) between the barrel and shaft attachment points and the pivot, mechanical advantage can be set to provide comfortable operation during strenuous bone cutting procedures. Additionally, the tendency of the forceps to move during actuation is reduced as the spacing 83 is increased while fine control is retained by keeping the grasped portion of the handle near the barrel and shaft attachment.

In operation (see Fig. 2) a bone segment 85 with an unsevered portion 87 is trapped between the shaft and plate 57 and the cutting edge 47 of the barrel. As the shaft is drawn into the barrel, severed bone 89 is captured within the barrel. Once the stroke has been completed the forceps are removed from the cutting site, inverted, and the shaft extended from the barrel to facilitate deposit of the severed bone into the proper receptacle.

When the shaft member is reciprocated to its full extent, as shown in Fig. 6, stop surface 37 of the barrel member and stop surface 63 of the plate member make full contact stopping the extent of reciprocation of the shaft member. At this point in the reciprocation, the two stop surfaces 37, 63 carry the full direct force applied to the handle members. Cutting edge 47 of the barrel member and the cutting edge 71 of the plate member are located relative to their associated stop surfaces 27, 63, respectively, so that cutting edges 47, 71 barely contact one another or, in fact, avoid contacting one another. This locating of the cutting edges avoids direct force contact of the cutting edges when the shaft member is reciprocated to its full extent. A major part of or the totality of the force applied on the handles is applied to the stop surfaces. This prevents the shag cutting edges 47, 71 from unnecessary waring.

While we have shown a presently preferred embodiment of the present invention, it will be apparent to those skilled in the art that the invention may be otherwise embodied within the scope of the appended claims.

## Claims

1. A medical instrument for cutting and removing bone (85) comprising :
a rigid barrel member (29) having a first working area (33) defined at one extremity thereof, said first working area including a first cutting surface (35) and a flat stop surface (37) ;
a shaft member (49) positioned coaxially within said barrel member and arranged for reciprocal motion therein, said shaft member having a plate member (57) mounted at one extremity thereof transverse to the axis of said shaft member and having a defined cavity (79) proximate to said plate member, said plate member having a second working area (59) comprising a second cutting surface (61) and a second flat stop surface (63), said first and second flat stop surfaces being disposed in a face confronting relationship and making full contact when said shaft member is reciprocated to its full extent within said barrel member, for stopping the extent of reciprocation of said shaft member, said first and second cutting surfaces being disposed in a cutting relation and substantially avoiding direct force contact when said shaft member is reciprocated to its said full extent within said barrel member ;
lever means (15) for reciprocally translating said shaft member within said barrel (a) to cause said plate member to reciprocate with respect to said barrel thereby trapping said bone between said first and second cutting surfaces and (b) to cause said cavity to reciprocate with respect to said barrel thereby capturing bone severed by said cutting surfaces within said cavity.

2. A medical instrument according to claim 1 wherein said first cutting surface is a first cutting edge (47).

3. A medical instrument according to claim 2 wherein said second cutting surface is a cutting edge (71).

4. A medical instrument according to claim 2 wherein said barrel member includes an outside face (41) forming the outer surface of said barrel member and an inside face (43) spaced from said outside face and defining the containing area of said barrel member, said first cutting surface including : said first cutting edge disposed on said outside face at a first portion of the extremity thereof ; and a bevel surface extending between said first cutting edge and said inside face of said barrel member.

5. A medical instrument according to claim 4 wherein said first flat stop surface is formed of a flat surface extending between a second portion of the extremity of said outside face and said inside face of said barrel member.

6. A medical instrument according to claim 5, wherein said flat surface is orthogonal to said outside face.

7. A medical instrument according to claim 6 wherein said barrel member includes a uniform cross-sectional shape at said one extremity of said barrel member, said shape being somewhat rectangular formed of three straight sides and a protruding curved fourth side.

8. A medical instrument according to claim 7 wherein said first cutting edge is disposed on said protruding curved fourth cross section of said barrel member.

9. A medical instrument according to claim 3 wherein said plate member includes an outside face (69) forming the outer surface of said plate member and an inside face (65) confronting said barrel member, said inside face includes a cavity (67) formed into said plate member to form said second cutting surface, said second cutting surface including a second cutting edge formed by the intersection of a portion of said outside face and a portion of the surface defining said cavity.

## Patentansprüche

1. Medizinisches Instrument zum Schneiden und Entfernen von Knochen (85), das aufweist :
ein starres Rohrglied (29) mit einem ersten Arbeitsbereich (33), der an einem Ende desselben ausgebildet ist, wobei der erste Arbeitsbereich eine erste Schneidfläche (35) und eine flache Anschlagfläche (37) aufweist ;

ein Schaftglied (49), das koaxial in dem Rohrglied angeordnet und für Hin- und Herbewegung in demselben ausgebildet ist, wobei das Schaftglied ein Plattenglied (57) aufweist, das an seinem einen Ende quer zur Achse des Schaftgliedes befestigt ist und das einen dem Plattenglied benachbarten abgegrenzten Hohlraum (79) aufweist, wobei das Plattenglied einen zweiten Arbeitsbereich (59) aufweist, der eine zweite Schneidfläche (61) und eine zweite flache Anschlagfläche (63) aufweist, wobei die ersten und zweiten flachen Anschlagflächen einander flächig gegenüberstehen und sich voll berühren, wenn das Schaftglied völlig in das Rohrglied zurückbewegt ist, um das Ausmaß der Zurückbewegung des Schaftglieds zu begrenzen, wobei die ersten und zweiten Schneidflächen in einer Schneidbeziehung angeordnet sind und im wesentlichen direkter Kraftkontakt vermieden wird, wenn das Schaftglied völlig in das Rohrglied zurückbewegt ist ;

Hebelmittel (15), um das Schaftglied innerhalb des Rohrs hin- und herzubewegen, um (a) zu bewirken, daß sich das Plattenglied in bezug auf das Rohr hin- und herbewegt, um dabei Knochen zwischen den ersten und zweiten Schneidflächen einzuschließen, und um (b) zu bewirken, daß sich der Hohlraum in bezug auf das Rohr hin- und herbewegt, um durch die Schneidflächen abgetrennten Knochen innerhalb des Hohlraumes aufzufangen.

2. Medizinisches Instrument nach Anspruch 1, bei dem die erste Schneidfläche eine erste Schneidkante (47) ist.

3. Medizinisches Instrument nach Anspruch 2, bei dem die zweite Schneidfläche eine Schneidkante (71) ist.

4. Medizinisches Instrument nach Anspruch 2, bei dem das Rohrglied eine Außenfläche (41), die die äußere Oberfläche des Rohrgliedes bildet, und eine Innenfläche (43) aufweist, die von der Außenfläche einen Abstand aufweist und den Aufnahmeraum des Rohrgliedes begrenzt, wobei die erste Schneidoberfläche die erste Schneidkante, die an der Außenfläche an einem ersten Bereich am Ende derselben angeordnet ist, und eine Schrägfläche aufweist, die sich zwischen der ersten Schneidkante und der Innenfläche des Rohrgliedes erstreckt.

5. Medizinisches Instrument nach Anspruch 4, bei dem die erste flache Anschlagfläche durch eine flache Oberfläche gebildet wird, die sich zwischen einem zweiten Bereich des Endes der Außenfläche und der Innenfläche des Rohrgliedes erstreckt.

6. Medizinisches Instrument nach Anspruch 5, bei dem die flache Oberfläche senkrecht zur Außenfläche steht.

7. Medizinisches Instrument nach Anspruch 6, bei dem das Rohrglied gleichförmige Querschnittsform an dem einen Ende des Rohrgliedes aufweist, wobei die Form in etwa rechteckig ist aus drei geraden Seiten und einer vorstehenden gekrümmten vierten Seite besteht.

8. Medizinisches Instrument nach Anspruch 7, bei dem die erste Schneidkante auf dem vorstehenden gekrümmten vierten Querschnitt des Rohrgliedes angeordnet ist.

9. Medizinisches Instrument nach Anspruch 3, bei dem das Plattenglied eine Außenfläche (69), die die äußere Oberfläche des Plattengliedes bildet, und eine Innenfläche (65) aufweist, die zum Rohrglied gerichtet ist, wobei die Innenfläche einen Hohlraum (67) einschließt, der in dem Plattenglied ausgebildet ist, um die zweite Schneidfläche zu bilden, wobei die zweite Schneidfläche eine zweite Schneidkante einschließt, die durch die Schnittlinie eines Teils der Außenfläche und eines Teils der Oberfläche, die den Hohlraum begrenzt, gebildet ist.

## Revendications

1. Instrument médical pour la coupe et l'excision d'os (85), comprenant :

un cylindre rigide (29) à l'une des extrémités duquel est définie une première zone de travail (33), cette zone de travail comprenant une première surface de coupe (35) et une surface d'arrêt (37) plate ;

une tige (49) disposée coaxialement dans ledit cylindre et agencée de façon à pouvoir y effectuer un mouvement de va-et-vient, cette tige comportant un élément en plaque (57) qui est monté à l'une de ses extrémités, perpendiculairement à l'axe de ladite tige, et présentant une cavité définie (79) contiguë audit élément en plaque, ledit élément en plaque présentant une seconde zone de travail (59) qui comprend une seconde surface de coupe (61) et une seconde surface d'arrêt (63) plate, lesdites première et seconde surfaces d'arrêt plates étant opposées l'une en face de l'autre et entrant entièrement en contact l'une avec l'autre lorsque ladite tige est rentrée sur toute son étendue dans ledit cylindre, afin de limiter le mouvement de va-et-vient de ladite tige, lesdites première et seconde surfaces de coupe étant disposées en rapport mutuel de coupe sans entrer pratiquement en contact direct forcé lorsque ladite tige est rentrée sur toute son étendue dans ledit cylindre :

des moyens à levier (15) pour produire un mouvement alternatif de translation de ladite tige dans ledit cylindre, (a) afin d'imprimer un mouvement de va-et-vient audit élément en plaque par rapport audit cylindre et piéger ainsi ledit os entre lesdites première et seconde surfaces de coupe et (b) afin d'imprimer un mouvement de va-et-vient à ladite cavité par rapport audit cylindre et capturer ainsi dans la cavité l'os sectionné par lesdites surfaces de coupe.

2. Instrument médical selon la revendication 1, dans lequel ladite première surface de coupe est une première arête tranchante (47).

3. Instrument médical selon la revendication 2, dans lequel ladite seconde surface de coupe est une arête tranchante (71).

4. Instrument médical selon la revendication 2, dans lequel ledit cylindre présente une face externe (41) formant, la surface extérieure dudit cylindre et une face interne (43) située à distance de ladite face externe et définissant la zone contenante dudit cylindre, ladite première surface de coupe comprenant ladite première arête tranchante située sur ladite face externe dans une première partie de l'extrémité de celle-ci, et une surface biseautée s'étendant entre ladite première arête tranchante et ladite face interne dudit cylindre.

5. Instrument médical selon la revendication 4, dans lequel ladite première surface d'arrêt plate est constituée par une surface plate s'étendant entre une seconde partie de l'extrémité de ladite face externe et ladite face interne dudit cylindre.

6. Instrument médical selon la revendication 5, dans lequel ladite surface plate est perpendiculaire à ladite face externe.

7. Instrument médical selon la revendication 6, dans lequel ledit cylindre a une section de forme uniforme au niveau de ladite extrémité, cette forme étant à peu près rectangulaire délimitée par trois côtés rectilignes et un quatrième côté courbe saillant.

8. Instrument médical selon la revendication 7, dans lequel ladite première arête tranchante est située sur ledit quatrième côté courbe saillant de la section dudit cylindre.

9. Instrument médical selon la revendication 3, dans lequel ledit élément en plaque comprend une face externe (69) constituant la surface extérieure dudit élément en plaque et une face interne (65) vis-à-vis dudit cylindre, cette face interne contenant une cavité (67) creusée dans ledit élément en plaque pour former ladite seconde surface de coupe, ladite seconde surface de coupe comprenant une seconde arête tranchante formée par l'intersection d'une partie de ladite face externe et d'une partie de la surface définissant ladite cavité.

Fig. 1

Fig. 2

*Fig. 4*

*Fig. 3*

EP 0 243 803 B1

**Fig.5**

**Fig.6**

**Fig.7**

**Fig.8**